# EUROPÄISCHE PATENTANMELDUNG

(11) **EP 4 671 230 A1**
(43) Veröffentlichungstag der Anmeldung: **31.12.2025**
(21) Anmeldenummer: 24185045.2
(22) Anmeldetag: 27.06.2024
(51) Int. Cl.: C07C 45/51, C07C 45/78, C07C 67/00, C07C 67/56

(54) **VERFAHREN ZUR HERSTELLUNG VON MONOAROMAT-DERIVATEN DURCH OXIDATIVE DEPOLYMERISIERUNG VON LIGNIN, POLYOXOMETALLAT-KATALYSATOR UND DESSEN VERWENDUNG BEI DEM VERFAHREN ZUR HERSTELLUNG VON MONOAROMAT-DERIVATEN**

(71) Anmelder: LigPOMMem GmbH, 90427 Nürnberg (DE); Universität Hamburg, 20148 Hamburg (DE)
(72) Erfinder: ALBERT, Jakob (Prof. Dr.-Ing. habil.), 22523 Hamburg (DE); PAPAJEWSKI, Max Philipp, 22523 Hamburg (DE); WESINGER, Stefanie, 20539 Hamburg (DE)
(74) Vertreter: Louis Pöhlau Lohrentz

(57) **Zusammenfassung**

Die vorliegende Erfindung betrifft ein Verfahren zur Herstellung von Monoaromat-Derivaten durch oxidative Depolymerisierung von Lignin, einen Polyoxometallat-Katalysator mit der allgemeinen Formel (I) sowie dessen Verwendung in einem Verfahren zur Herstellung von Monoaromat-Derivaten mit abschließender Abtrennung der Monoaromaten durch Membrantrennung

## Beschreibung

Die vorliegende Erfindung betrifft ein Verfahren zur Herstellung von Monoaromat-Derivaten durch oxidative Depolymerisierung von Lignin, einen Polyoxometallat-Katalysator mit der allgemeinen Formel (I) sowie dessen Verwendung in einem Verfahren zur Herstellung von Monoaromat-Derivaten.

Derzeit erfolgt die Produktion von niedermolekularen Aromaten hauptsächlich durch Umsetzung von petrochemisch hergestellten Grundbausteinen, zum Beispiel Benzol, welches nachfolgend derivatisiert wird um beispielsweise Guajacol herzustellen.

Lignine bilden eine Gruppe von phenolischen Makromolekülen, die sich aus verschiedenen, aromatischen Monomerbausteinen zusammensetzen. Etwa 20 bis 30 Prozent der Trockenmasse verholzter Pflanzen bestehen aus Ligninen, die damit neben der Cellulose und dem Chitin die häufigsten organischen Verbindungen der Erde darstellt.

Darüber hinaus fallen beispielsweise rund 50 Millionen Tonnen Lignin jährlich weltweit als Abfallprodukt der Papierindustrie an. Um Zellstoff aus Holz zu gewinnen, nutzt man bei der Papierherstellung häufig das Sulfatverfahren. Beim Sulfatverfahren kocht man entrindete Holzschnitzel, Stroh oder zerkleinerte Maisstängel unter Druck mehrere Stunden in Natronlauge, um das Lignin von der faserigen Cellulose zu lösen. Das stoffliche Potenzial von Lignin, das bei diesem Verfahren als Schwarzlauge anfällt, bleibt weitgehend ungenutzt, da 98 Prozent des anfallenden Lignins verbrannt werden.

Da Lignine viele aromatische, vorzugsweise monoaromatische, Gruppen aufweisen, haben Lignine das Potenzial, petrochemische Rohstoffe bei der Herstellung von monoaromatischen Derivaten zu ersetzen. Allerdings gibt es bislang noch kein industrielles Verfahren, das für die Gewinnung von ausreichenden Mengen von monoaromatischen Produkten aus Lignin verwendet wird.

Aus der US 5,695,606, der US 5,302,248 und der US 5,552,019 sind Verfahren zum oxidativen Abbau von Lignozellulose unter Erhalt der Zellulose bekannt, wobei die Verfahren zur Abtrennung von Zellstoff von unerwünschtem Lignin dienen.

Aus der WO 2012/034839 A1 ist ein Verfahren zur katalytischen Erzeugung von Ameisensäure mit Polyoxometallaten bekannt, wobei ein vollständiger Abbau des Lignins zu Ameisensäure und Kohlendioxid stattfindet.

In einem aus Bjorsvik, H.R und Minisci, F. (1999) (Fine Chemicals from Lignosulfonates. 1. Synthesis of Vanillin by Oxidation of Lignosulfonates, Org. Process Res. Dev. 3(5), 1999, Seiten 330-340) bekannten Verfahren wird Vanillin durch oxidativen Lignin-Abbau hergestellt, wobei der Abbau jedoch etwa 10 mol Sauerstoff und 27 bis 53 mol einer Base, vorzugsweise NaOH, pro 1 mol Vanillin verbraucht. Bei dem verwendeten Verfahren kann nur Lignosulfonat verwendet werden.

Es ist daher die Aufgabe der vorliegenden Erfindung ein Verfahren bereitzustellen, dass eine effiziente, vorzugsweise kontinuierliche, Produktion von Monoaromaten, wie beispielsweise Vanillin, Methylvanillat, Syringaldehyd, und/oder Methylsyringat, aus zahlreichen industriellen, industriell verfügbaren ligninhaltigen Produkten, vorzugsweise aus Kraft-Lignin, Organosolv-Lingnin und/oder Lignosulfonat, ermöglicht.

Die Aufgabe der vorliegenden Erfindung wird gelöst durch die Bereitstellung eines Verfahrens gemäß Anspruch 1 zur Herstellung von Monoaromat-Derivaten durch oxidative Depolymerisierung von Lignin, wobei das Verfahren folgende Schritte umfasst:
(1a) Bereitstellen einer Lignin-haltigen Zusammensetzung,
(1b) Kontaktieren der Lignin-haltigen Zusammensetzung mit Sauerstoff in Gegenwart wenigstens eines Polyoxometallat - Katalysators mit der allgemeinen Formel Aₙ[XY_{y}Z_{z}MₘO₄₀],
wobei A Wasserstoff oder ein Gegenion, das ausgewählt ist aus Alkalimetall, oder Ammonium, bedeutet, wobei n eine ganze Zahl aus einem Bereich von 6 bis 15 bedeutet, und
wobei X ein Heteroatom bedeutet, das ausgewählt ist aus Phosphor oder Silizium, vorzugsweise Phosphor,
wobei Y und Z jeweils voneinander verschieden sind und ein Metallatom bedeuten, das jeweils unabhängig voneinander ausgewählt ist aus, Nickel (Ni), Niob (Nb), Indium (In) und Cobalt (Co), und
wobei M ein Gerüstatom bedeutet, das ausgewählt ist aus der Gruppe, die aus Molybdän (Mo), Wolfram (W) und Kombinationen davon besteht,
wobei der Index m eine ganze Zahl aus einem Bereich von 6 bis 11 bedeutet, wobei der Index y eine ganze Zahl aus einem Bereich von 0 bis 4 bedeutet, und wobei der Index z eine ganze Zahl aus einem Bereich von 1 bis 4 bedeutet bedeuten, mit der Maßgabe, dass die Summe der Indices m, y und z gleich 12 ist,
wobei das Kontaktieren der Lignin-haltigen Zusammensetzung mit Sauerstoff in Gegenwart des wenigstens einen Polyoxometallat - Katalysators in einem Alkohol-haltigen Lösungsmittel, das Methanol und/oder Ethanol und/oder n-Propanol umfasst oder daraus besteht, bei einer Temperatur von unter 160 °C und in Gegenwart von Sauerstoff mit einem Sauerstoffpartialdruck von höchstens 30 bar erfolgt, und
(1c) Abtrennen der gebildeten Monoaromat-Derivate,
wobei die Monoaromat-Derivate ausgewählt sind aus der Gruppe, die aus Vanillin, Isovanillin, *ortho*-Vanillin, Acetovanillon, Vanillinsäure-Alkylester, Syringaldehyd, Syringasäure-Alkylester, Acetosyringon, Hydroxybenzaldehyd, Hydroxybenzoesäure-Alkylester, 4-Hydroxyacetophenon und Mischungen davon, vorzugsweise Vanillin, Vanillinsäure-methylester, Vanillinsäure-ethylester, Vanillinsäure-n-propylester Syringaldehyd, Syringasäure-methylester, Syringasäure-ethylester, Syringasäure-n-propylester, und Mischungen davon, besteht.

Bevorzugte Ausführungsformen des erfindungsgemäßen Verfahrens werden in den abhängigen Ansprüchen 2 bis 11 und 14 aufgeführt.

Die Aufgabe der vorliegenden Erfindung wird weiterhin gelöst durch die Bereitstellung eines Polyoxometallat - Katalysators gemäß Anspruch 12, wobei der Polyoxometallat - Katalysator die allgemeine Formel (I):

Aₙ[XY_{y}Z_{z}MₘO₄₀] (I)

aufweist,
wobei A Wasserstoff oder ein Gegenion, das ausgewählt ist aus Alkalimetall, oder Ammonium, bedeutet, wobei n eine ganze Zahl aus einem Bereich von 6 bis 15 bedeutet, und
wobei X ein Heteroatom bedeutet, das ausgewählt ist aus Phosphor oder Silizium, vorzugsweise Phosphor,
wobei Y und Z jeweils voneinander verschieden sind und ein Metallatom bedeuten, das jeweils unabhängig voneinander ausgewählt ist aus, Nickel (Ni), Niob (Nb), Indium (In) und Cobalt (Co), und
wobei M ein Gerüstatom bedeutet, das ausgewählt ist aus der Gruppe, die aus Molybdän (Mo), Wolfram (W) und Kombinationen davon besteht,
wobei der Index m eine ganze Zahl aus einem Bereich von 6 bis 11 bedeutet, wobei der Index y eine ganze Zahl aus einem Bereich von 0 bis 4 bedeutet, und wobei der Index z eine ganze Zahl aus einem Bereich von 1 bis 4 bedeutet bedeuten, mit der Maßgabe, dass die Summe der Indices m, y und z gleich 12 ist.

Bevorzugte Ausführungsformen des erfindungsgemäßen Polyoxometallat - Katalysators werden im abhängigen Anspruch 13 aufgeführt.

Bei dem erfindungsgemäßen Verfahren gemäß Anspruch 1 zur Herstellung von Monoaromat-Derivaten durch oxidative Depolymerisierung von Lignin, wird in Schritt (1a) zunächst eine Lignin-haltige Zusammensetzung bereitgestellt, die vorzugsweise wenigstens ein Organosolv-Lignin, wenigstens ein Kraft-Lignin und/oder wenigstens ein Lignosulfonat umfasst.

Unter dem Begriff "Ligninsulfonate" oder auch "Lignosulfonate" werden vorzugsweise die Salze der Ligninsulfonsäure, einem wasserlöslichen, anionischen, polyelektrolytischen, verzweigten Polymer verstanden. Sie sind Nebenprodukte bei der Herstellung von Zellstoff mit dem Sulfitverfahren.

Vorzugsweise umfasst die in Schritt (1a) bereitgestellte Lignin-haltige Zusammensetzung weiterhin ein Alkohol-haltiges Lösungsmittel, das Methanol und/oder Ethanol und/oder n-Propanol umfasst oder daraus besteht, wobei zumindest ein Teil des in der Lignin-haltigen Zusammensetzung enthaltene Lignin und/oder Lingnosulfonat in dem Alkohol-haltigen Lösungsmittel gelöst vorliegt.

Weiter bevorzugt weist die in Schritt (1a) bereitgestellte Lignin-haltige Zusammensetzung wenigstens eine, vorzugsweise feststofffreie, Flüssigphase auf.

Bei einer bevorzugten Ausführungsform umfasst die in Schritt (1a) bereitgestellte Lignin-haltige Zusammensetzung wenigstens ein Lignin und/oder Lingnosulfonat, das unter Verwendung von einem Hartholz, das vorzugsweise eine Darrdichte von über 0,55 g/cm³ aufweist, beispielsweise Buche, Eiche, und/oder Esche, erhalten wurde.

Unter dem Begriff "Darrdichte" wird vorzugsweise die durchschnittliche Rohdichte von trockenem Holz, das heißt bei 0 % Holzfeuchte, verstanden. Die Darrdichte kann beispielsweise gemäß der in der DIN 52182 - 1976-09 ("Prüfung von Holz; Bestimmung der Rohdichte", Ausgabedatum: 09.1976) beschriebenen Verfahren bestimmt werden.

Die in Schritt (1a) bereitgestellte Lignin-haltige Zusammensetzung wird in Schritt (1b) des erfindungsgemäßen Verfahrens mit Sauerstoff in Gegenwart wenigstens eines Polyoxometallat - Katalysators mit der allgemeinen Formel (I):

Aₙ[XY_{y}Z_{z}MₘO₄₀] (I)

kontaktiert, wobei A Wasserstoff oder ein Gegenion, das ausgewählt ist aus Alkalimetall-Kation, oder Ammonium, bedeutet, wobei n eine ganze Zahl aus einem Bereich von 6 bis 15 bedeutet, und wobei X ein Heteroatom bedeutet, das ausgewählt ist aus Phosphor oder Silizium, vorzugsweise Phosphor, wobei Y und Z jeweils voneinander verschieden sind und ein Metallatom bedeuten, das jeweils unabhängig voneinander ausgewählt ist aus, Nickel (Ni), Niob (Nb), Indium (In) und Cobalt (Co), und wobei M ein Gerüstatom bedeutet, das ausgewählt ist aus der Gruppe, die aus Molybdän (Mo), Wolfram (W) und Kombinationen davon besteht, wobei der Index m eine ganze Zahl aus einem Bereich von 6 bis 11 bedeutet, wobei der Index y eine ganze Zahl aus einem Bereich von 0 bis 4 bedeutet, und wobei der Index z eine ganze Zahl aus einem Bereich von 1 bis 4 bedeutet bedeuten, mit der Maßgabe, dass die Summe der Indices m, y und z gleich 12 ist, wobei das Kontaktieren der Lignin-haltigen Zusammensetzung mit Sauerstoff in Gegenwart des wenigstens einen Polyoxometallat - Katalysators in einem Alkohol-haltigen Lösungsmittel, das Methanol und/oder Ethanol und/oder n-Propanol umfasst oder daraus besteht, bei einer Temperatur von unter 160 °C in Gegenwart von Sauerstoff mit einem Sauerstoffpartialdruck von höchstens 30 bar erfolgt.

Vorzugsweise erfolgt das Kontaktieren in Schritt (1b) bei einer Temperatur aus einem Bereich von 50°C bis unter 160°C, vorzugsweise von 60°C bis 150°C, weiter bevorzugt von 90°C bis 140°C, weiter bevorzugt von 120°C bis 140°C.

Die Erfinder haben festgestellt, dass oberhalb einer Temperatur von 160°C eine unerwünschte Plastifizierung von Lignin zu sog. "Pseudo-lignin" beziehungsweise eine unerwünschte Bildung von Huminen auftritt.

Der in Schritt (1b) des erfindungsgemäßen Verfahrens verwendete Sauerstoff kann vorzugsweise in Form von reinem Sauerstoff oder in Form von Luft verwendet werden, wobei der Sauerstoffpartialdruck höchstens 30 bar beträgt. Weiter bevorzugt erfolgt das Kontaktieren in Schritt (1b) des erfindungsgemäßen Verfahrens bei einem Sauerstoffpartialdruck aus einem Bereich von 1 bar bis 30 bar, vorzugsweise von 5 bar bis 28 bar, weiter bevorzugt von 10 bar bis 25 bar, weiter bevorzugt von 12 bar bis 18 bar.

Weiter bevorzugt kann durch Kontaktieren der in Schritt (1a) bereitgestellten Ligninhaltigen Zusammensetzung in Schritt (1b) des erfindungsgemäßen Verfahrens mit Sauerstoff bei einem Sauerstoffpartialdruck von höchstens 30 bar in Gegenwart wenigstens eines Polyoxometallat - Katalysators bei der oben angegeben Temperatur von unter 160 °C ein weiterer oxidativer Abbau der gebildeten Monoaromat-Derivate, insbesondere Vanillin, Isovanillin, ortho-Vanillin, Acetovanillon, Vanillinsäure-C1-C3-Alkylester, Syringaldehyd, Syringasäure-C1-C3-Alkylester, Acetosyringon, Hydroxybenzaldehyd, Hydroxybenzoesäure-C1-C3-Alkylester, 4-Hydroxyacetophenon und Mischungen davon, verringert werden.

Die Erfinder haben festgestellt, dass der verwendete Sauerstoffpartialdruck von höchstens 30 bar dabei ein ausgewogenes Verhältnis von gewünschtem Ligninumsatz und unerwünschter vollständiger Oxidation der gebildeten Monoaromat-Derivate zu CO₂ und CO ermöglicht.

Weiterhin haben die Erfinder festgestellt, dass vorzugsweise durch Änderung des Sauerstoffpartialdrucks, bei dem Schritt (1b) des erfindungsgemäßen Verfahrens durchgeführt wird und der 30 bar nicht überschreitet, die Verteilung der erhaltenen Monoaromaten, vorzugsweise Vanillin, Isovanillin, ortho-Vanillin, Acetovanillon, Vanillinsäure-C1-C3-Alkylester, Syringaldehyd, Syringasäure-C1-C3-Alkylester, Acetosyringon, Hydroxybenzaldehyd, Hydroxybenzoesäure-C1-C3-Alkylester, und 4-Hydroxyacetophenon, variiert werden kann.

Vorzugsweise wird ein Vanillinsäure-C1-C3-Alkylester aus der Gruppe, die aus Vanillinsäure-methylester, Vanillinsäure-ethylester, Vanillinsäure-n-propylester und Mischungen davon besteht, ausgewählt.

Vorzugsweise wird ein Syringasäure-C1-C3-Alkylester aus der Gruppe, die aus Syringasäure-methylester, Syringasäure-ethylester, Syringasäure-n-propylester und Mischungen davon besteht, ausgewählt.

Vorzugsweise wird ein Hydroxybenzoesäure-C1-C3-Alkylester aus der Gruppe, die aus Hydroxybenzoesäure-methylester, Hydroxybenzoesäure-ethylester, Hydroxybenzoesäure-n-propylester und Mischungen davon besteht, ausgewählt.

Der in Schritt (1b) verwendete Polyoxometallat - Katalysator weist die allgemeine Formel (I):

Aₙ[XY_{y}Z_{z}MₘO₄₀] (I)

auf, wobei A Wasserstoff oder ein Gegenion, das ausgewählt ist aus Alkalimetall-Kationen, vorzugsweise Na⁺, K⁺ oder Li⁺, oder Ammonium (NH₄⁺), bedeutet, wobei n eine ganze Zahl aus einem Bereich von 6 bis 15 bedeutet, und wobei X ein Heteroatom bedeutet, das ausgewählt ist aus Phosphor oder Silizium, vorzugsweise Phosphor, wobei Y und Z jeweils voneinander verschieden sind und ein Metallatom bedeuten, das jeweils unabhängig voneinander ausgewählt ist aus, Nickel (Ni), Niob (Nb), Indium (In) und Cobalt (Co), und wobei M ein Gerüstatom bedeutet, das ausgewählt ist aus der Gruppe, die aus Molybdän (Mo), Wolfram (W) und Kombinationen davon besteht, wobei der Index m eine ganze Zahl aus einem Bereich von 6 bis 11 bedeutet, wobei der Index y eine ganze Zahl aus einem Bereich von 0 bis 4 bedeutet, und wobei der Index z eine ganze Zahl aus einem Bereich von 1 bis 4 bedeutet bedeuten, mit der Maßgabe, dass die Summe der Indices m, y und z gleich 12 ist.

Vorzugsweise wird der verwendete Polyoxometallat - Katalysator mit der allgemeinen Formel (I) in Schritt (1b) in einem Substrat zu Katalysator-Verhältnis aus einem Bereich von 15:1 bis 1:1, vorzugsweise von 10 : 1 bis 1 : 1, vorzugsweise von 8 : 1 bis 2 :1, weiter bevorzugt von 6 : 1 bis 3: 1, jeweils bezogen auf das Verhältnis des Gesamtgewicht des verwendeten Katalysators zu dem eingesetzten Gesamtgewicht des verwendeten Lignins und/oder Lignosulfonats.

Weiter bevorzugt wird der in Schritt (1b) verwendete Polyoxometallat - Katalysator mit der allgemeinen Formel (I) aus der Gruppe, die aus H₁₅PCo₃Mo₉O₄₀, H₁₁PCo₂Mo₁₀O₄₀, H₁₅PNi₃Mo₉O₄₀, H₁₁PNi₂Mo₁₀O₄₀, H₁₁PNiCoMo₁₀O₄₀ und Mischungen davon besteht, ausgewählt.

Ein erfindungsgemäßer Polyoxometallat - Katalysator weist die allgemeine Formel (I):

Aₙ[XY_{y}Z_{z}MₘO₄₀] (I)

auf, wobei A Wasserstoff oder ein Gegenion, das ausgewählt ist aus Alkalimetall, oder Ammonium, bedeutet, wobei n eine ganze Zahl aus einem Bereich von 6 bis 15 bedeutet, und wobei X ein Heteroatom, das ausgewählt ist aus Phosphor oder Silizium, vorzugsweise Phosphor, bedeutet, und wobei Y und Z jeweils voneinander verschieden sind und ein Metallatom bedeuten, das jeweils unabhängig voneinander ausgewählt ist aus, Nickel (Ni), Niob (Nb), Indium (In) und Cobalt (Co), und wobei M ein Gerüstatom bedeutet, das ausgewählt ist aus der Gruppe, die aus Molybdän (Mo), Wolfram (W) und Kombinationen davon besteht, wobei der Index m eine ganze Zahl aus einem Bereich von 6 bis 11 bedeutet, wobei der Index y eine ganze Zahl aus einem Bereich von 0 bis 4 bedeutet, und wobei der Index z eine ganze Zahl aus einem Bereich von 1 bis 4 bedeutet bedeuten, mit der Maßgabe, dass die Summe der Indices m, y und z gleich 12 ist.

Bei einem erfindungsgemäßen Polyoxometallat - Katalysator mit der allgemeinen Formel (I) und/oder einem erfindungsgemäß verwendeten Polyoxometallat - Katalysator mit der allgemeinen Formel (I) handelt es sich vorzugsweise um einen Heteropolyanion, das protoniert sein kann oder Gegenionen, die ausgewählt sind aus Alkalimetall-Kationen, vorzugsweise Na⁺, K⁺ oder Li⁺, oder Ammonium (NH₄⁺), aufweisen kann.

Ein erfindungsgemäßer Polyoxometallat - Katalysator mit der allgemeinen Formel (I) und/oder ein erfindungsgemäß verwendeter Polyoxometallat - Katalysator mit der allgemeinen Formel (I) weist vorzugsweise eine Keggin-Struktur auf, wobei weiter bevorzugt A Wasserstoff oder ein Gegenion, das ausgewählt ist aus Alkalimetall-Kation, weiter bevorzugt Na⁺, K⁺ oder Li⁺, oder Ammonium, bedeutet, wobei n eine ganze Zahl aus einem Bereich von 6 bis 15 bedeutet, und wobei X ein Heteroatom, das ausgewählt ist aus Phosphor oder Silizium, vorzugsweise Phosphor, bedeutet, und wobei Y und Z jeweils voneinander verschieden sind und ein Metallatom bedeuten, das jeweils unabhängig voneinander ausgewählt ist aus, Nickel (Ni), Niob (Nb), Indium (In) und Cobalt (Co), vorzugsweise Nickel (Ni), und Cobalt (Co), und wobei M ein Gerüstatom bedeutet, das ausgewählt ist aus der Gruppe, die aus Molybdän (Mo), Wolfram (W) und Kombinationen davon, vorzugsweise Molybdän (Mo), besteht, wobei der Index m eine ganze Zahl aus einem Bereich von 6 bis 11 bedeutet, wobei der Index y eine ganze Zahl aus einem Bereich von 0 bis 4 bedeutet, und wobei der Index z eine ganze Zahl aus einem Bereich von 1 bis 4 bedeutet bedeuten, mit der Maßgabe, dass die Summe der Indices m, y und z gleich 12 ist.

Das Kontaktieren der in Schritt (1a) bereitgestellten Lignin-haltigen Zusammensetzung in Schritt (1b) des erfindungsgemäßen Verfahrens mit Sauerstoff mit einem Sauerstoffpartialdruck von höchstens 30 bar in Gegenwart wenigstens eines Polyoxometallat - Katalysators bei der oben angegeben Temperatur von unter 160 °C erfolgt in einem Alkohol-haltigen Lösungsmittel, das wenigstens einen aliphatischen Alkohol mit 1 bis 3 Kohlenstoff-Atomen, vorzugsweise 1 bis 2 Kohlenstoff-Atomen, und 1 OH-Gruppe, vorzugsweise Methanol und/oder Ethanol und/oder n-Propanol, vorzugsweise Methanol und/oder Ethanol, umfasst oder daraus besteht.

Das in Schritt (1b) verwendete Alkohol-haltige Lösungsmittel umfasst vorzugsweise weiterhin Wasser in einem Anteil von mindestens 0,5 Vol.-% bis höchstens 40 Vol.- %, vorzugsweise von mindestens 5 Vol.-% bis höchstens 35 Vol.-%, jeweils bezogen auf das Gesamtvolumen des Alkohol-haltigen Lösungsmittels.

Das in Schritt (1b) verwendete Alkohol-haltige Lösungsmittel ist weiter bevorzugt ein Alkohol-Wasser-Gemisch, das wenigstens einen aliphatischen Alkohol mit 1 bis 3 Kohlenstoff-Atomen, vorzugsweise 1 bis 2 Kohlenstoff-Atomen, und 1 OH-Gruppe, vorzugsweise Methanol und/oder Ethanol und/oder n-Propanol, vorzugsweise Methanol und/oder Ethanol, und Wasser in einem Anteil von mindestens 0,5 Vol.-% bis höchstens 40 Vol.-%, vorzugsweise von mindestens 5 Vol.-% bis höchstens 35 Vol.-%, jeweils bezogen auf das Gesamtvolumen des Alkohol-haltigen Lösungsmittels, umfasst oder daraus besteht.

Durch Wahl des jeweils in Schritt (1b) verwendeten aliphatischen Alkohols kann weiterhin die chemische Zusammensetzung der gebildeten Monoaromat-Derivate, insbesondere der Vanillinsäure-C1-C3-Alkylester und/oder Syringasäure-C1-C3-Alkylester beeinflusst werden, wobei bei Verwendung von Methanol vorzugsweise neben Vanillin, und Syringaldehyd, Vanillinsäure-methylester und Syringasäure-methylester gebildet werden, während bei Verwendung von Ethanol vorzugsweise neben Vanillin, und Syringaldehyd, Vanillinsäure-ethylester und Syringasäure-ethylester gebildet werden.

Bei Verwendung von n-Propanol wird vorzugsweise neben Vanillin, und Syringaldehyd, Vanillinsäure-n-propylester und Syringasäure-n-propylester gebildet.

Die Erfinder haben ebenfalls festgestellt, dass bei Verwendung eines Alkohol-haltigen Lösungsmittels, das wenigstens einen aliphatischen Alkohol mit 1 bis 3 Kohlenstoff-Atomen, vorzugsweise 1 bis 2 Kohlenstoff-Atomen, und 1 OH-Gruppe, vorzugsweise Methanol und/oder Ethanol und/oder n-Propanol, vorzugsweise Methanol und/oder Ethanol, und optional Wasser in einem Anteil von mindestens 0,5 Vol.-% bis höchstens 40 Vol.-%, vorzugsweise von mindestens 5 Vol.-% bis höchstens 35 Vol.-%, jeweils bezogen auf das Gesamtvolumen des Alkohol-haltigen Lösungsmittels, umfasst oder daraus besteht, in Schritt (1b) des erfindungsgemäßen Verfahrens sowohl das verwendete Lignin und/oder Lignosulfonat als auch der verwendete Polyoxometallat - Katalysator mit der allgemeinen Formel (I) in ausreichender Menge im verwendeten Alkohol-haltigen Lösungsmittel gelöst ist, damit eine ausreichende Umsetzung des Lignins und/oder Lignosulfonats mit Sauerstoff unter Erhalt der jeweiligen Monoaromat-Derivat erfolgen kann.

Vorzugsweise erfolgt das Kontaktieren der Lignin-haltigen Zusammensetzung mit Sauerstoff in Gegenwart des wenigstens einen Polyoxometallat - Katalysators in Schritt (1b) für wenigstens 0,5 h, vorzugsweise für wenigstens 2 h, vorzugsweise für einen Zeitraum von 2 h bis 36 h, weiter bevorzugt von 6 h bis 24 h.

Vorzugsweise kann während des Kontaktierens der Lignin-haltigen Zusammensetzung mit Sauerstoff in Schritt (1b) die Lignin-haltige Zusammensetzung, der wenigstens eine Polyoxometallat - Katalysators mit der allgemeinen Formel (I) und das Alkohol-haltige Lösungsmittel durch Mischen der Einzelkomponenten oder durch Einwirkung von physikalischer Kraft, beispielsweise durch Verwendung eines Rührwerks und/oder durch Einwirkung von Ultraschall, Kavitation, etwa in einem Rotor-Stator-System, vermischt werden.

Vorzugsweise können zusätzlich geeignete Additive, die vorzugsweise den pH-Wert regulieren, beispielsweise Puffersubstanzen, vorzugsweise Natriumhydrogenphosphat und/oder Natriumdihydrogenphosphat sowie die analogen Kaliumsalze, verwendet werden, um zu gewährleisten, dass zumindest während Schritt (1b) kein Feststoff ausfällt.

Weiter bevorzugt erfolgt das Kontaktieren der Lignin-haltigen Zusammensetzung mit Sauerstoff in Gegenwart des wenigstens einen Polyoxometallat - Katalysators in Schritt (1b) ohne Verwendung eines Lösungspromoters und/oder Beschleunigungspromotors, weiter bevorzugt ohne Verwendung von Natronlauge, Nitrobenzol, Schwefelsäure, Salzsäure, Salpetersäure, Kupfersulfat oder Toluolsulfonsäure.

In Schritt (1c) des erfindungsgemäßen Verfahrens erfolgt ein Abtrennen der gebildeten Monoaromat-Derivate, vorzugsweise durch den Fachmann bekannte Verfahren, wie weiter bevorzugt Flüssig-Flüssig-Extraktion und/oder Membrantrennung, vorzugsweise Nano-Membranfiltration.

Das in Schritt (1c) abgetrennte Monoaromat-Derivat ist ausgewählt aus der Gruppe, die aus Vanillin, Isovanillin, ortho-Vanillin, Acetovanillon, Vanillinsäure-C1-C3-Alkylester, Syringaldehyd, Syringasäure-C1-C3-Alkylester, Acetosyringon, Hydroxybenzaldehyd, Hydroxybenzoesäure-C1-C3-Alkylester, 4-Hydroxyacetophenon und Mischungen davon, vorzugsweise Vanillin, Vanillinsäure-methylester, Vanillinsäure-ethylester, Vanillinsäure-n-propylester Syringaldehyd, Syringasäure-methylester, Syringasäure-ethylester, Syringasäure-n-propylester, und Mischungen davon.

Weiter bevorzugt wird in Schritt (1b) des erfindungsgemäßen Verfahrens ein Alkohol-haltiges Lösungsmittel verwendet, wobei das Alkohol-haltige Lösungsmittel Methanol umfasst oder daraus besteht und wobei in Schritt (1c) des erfindungsgemäßen Verfahrens weiter bevorzugt Vanillin, Syringaldehyd, Vanillinsäure-methylester und Syringasäure-methylester abgetrennt werden, oder wobei das Alkohol-haltige Lösungsmittel Ethanol umfasst oder daraus besteht und wobei in Schritt (1c) des erfindungsgemäßen Verfahrens weiter bevorzugt Vanillin, Syringaldehyd, Vanillinsäure-ethylester und Syringasäure-ethylester abgetrennt werden, oder wobei das Alkohol-haltige Lösungsmittel n-Propanol umfasst oder daraus besteht und wobei in Schritt (1c) des erfindungsgemäßen Verfahrens weiter bevorzugt Vanillin, Syringaldehyd, Vanillinsäure-n-propylester und Syringasäure-n-propylester abgetrennt werden.

Geeignete Membranen zur Verwendung in Schritt (1c) des erfindungsgemäßen Verfahrens sind dem Fachmann bekannt und können beispielsweise kommerziell erworben werden. Weiter bevorzugt ist eine geeignete Membran zur Verwendung in Schritt (1c) des erfindungsgemäßen Verfahrens wenigstens eine Nanofiltrationsmembran mit einer Porengröße von höchstens 2 nm.

Geeignete Membranen sind beispielsweise Polypiperazin-basierte Nanofiltrationsmembranen der Fa. Mann + Hummel (Ludwigsburg, DE), die unter der Typenbezeichnung TRISEPO UA60 und TRISEPO XN45 kommerziell erhältlich sind, oder Polyethersulfone (PES)-basierte Membranen der Fa. Mann + Hummel, die unter der Typenbezeichnung NADIRO NP030 P kommerziell erhältlich sind.

Die Aufgabe der vorliegenden Erfindung wird ebenfalls gelöst durch die Bereitstellung einer Verwendung eines Polyoxometallat - Katalysators mit der allgemeinen Formel (I):

Aₙ[XY_{y}Z_{z}MₘO₄₀] (I),

wobei A Wasserstoff oder ein Gegenion, das ausgewählt ist aus Alkalimetall-Kation, weiter bevorzugt Na⁺, K⁺ oder Li⁺, oder Ammonium, bedeutet, wobei n eine ganze Zahl aus einem Bereich von 6 bis 15 bedeutet, und wobei X ein Heteroatom, das ausgewählt ist aus Phosphor oder Silizium, vorzugsweise Phosphor, bedeutet, und wobei Y und Z jeweils voneinander verschieden sind und ein Metallatom bedeuten, das jeweils unabhängig voneinander ausgewählt ist aus, Nickel (Ni), Niob (Nb), Indium (In) und Cobalt (Co), vorzugsweise Nickel (Ni), und Cobalt (Co), und wobei M ein Gerüstatom bedeutet, das ausgewählt ist aus der Gruppe, die aus Molybdän (Mo), Wolfram (W) und Kombinationen davon, vorzugsweise Molybdän (Mo), besteht, wobei der Index m eine ganze Zahl aus einem Bereich von 6 bis 11 bedeutet, wobei der Index y eine ganze Zahl aus einem Bereich von 0 bis 4 bedeutet, und wobei der Index z eine ganze Zahl aus einem Bereich von 1 bis 4 bedeutet bedeuten, mit der Maßgabe, dass die Summe der Indices m, y und z gleich 12 ist, zur Herstellung von, vorzugsweise Methoxy-substituierten, Monoaromat-Derivaten durch oxidative Depolymerisierung von Lignin.

Vorzugsweise kann die erfindungsgemäße Verwendung und das erfindungsgemäße Verfahren zur Herstellung von Methoxy-substituierte Monoaromat-Derivaten, die weiter bevorzugt ausgewählt werden aus der Gruppe, die aus Vanillin, Isovanillin, ortho-Vanillin, Acetovanillon, Vanillinsäure-C1-C3-Alkylester, Syringaldehyd, Syringasäure-C1-C3-Alkylester, Acetosyringon und Mischungen davon, vorzugsweise Vanillin, Vanillinsäure-methylester, Vanillinsäure-ethylester, Vanillinsäure-n-propylester Syringaldehyd, Syringasäure-methylester, Syringasäure-ethylester, Syringasäure-n-propylester, und Mischungen davon, besteht, und von Hydroxy-substituierten Monoaromat-Derivaten, die keine Methoxy-Gruppen aufweisen und vorzugsweise ausgewählt werden aus der Gruppe, die aus Hydroxybenzaldehyd, 4-Hydroxyacetophenon, Hydroxybenzoesäure-C1-C3-Alkylester, und Mischungen davon, weiter bevorzugt Hydroxybenzaldehyd, 4-Hydroxyacetophenon, Hydroxybenzoesäure-methylester, Hydroxybenzoesäure-ethylester, Hydroxybenzoesäure-n-propylester und Mischungen davon, besteht, verwendet werden.

Weiter bevorzugt wird der verwendete Polyoxometallat - Katalysator mit der allgemeinen Formel (I) aus der Gruppe, die aus H₁₅PCo₃Mo₉O₄₀, H₁₁PCo₂Mo₁₀O₄₀, Hi₅PNi₃Mo₉O₄₀, H₁₁PNi₂Mo₁₀O₄₀, H₁₁PNiCoMo₁₀O₄₀ und Mischungen davon besteht, ausgewählt.

Mit dem erfindungsgemäßen Verfahren ist der Abbau von ligninhaltigen Zwischenprodukten aus der Zellstoff- und Papierindustrie zu wertvollen Plattformchemikalien für diverse Branchen, beispielsweise Nahrungsmittelindustrie oder Pharmaindustrie, möglich.

Weiter bevorzugt wird bei dem erfindungsgemäßen Verfahren wenigstens ein erfindungsgemäßer Polyoxometallat - Katalysator mit der allgemeinen Formel (I) verwendet.

Vorzugsweise soll die energetische Verwertung der ligninhaltigen Zwischenprodukte durch die oxidative Depolymerisierung gemäß der vorliegenden Erfindung ersetzt werden.

Nachfolgend wird die Erfindung anhand von Beispielen und Figuren näher erläutert, ohne hierauf beschränkt zu sein.

### Figurenverzeichnis

Fig. 1 zeigt die in Beispiel 4.1 gemessene Ausbeute an Monoaromaten mit unterschiedlichen Lösungsmittelgemischen.
Fig. 2 zeigt die in Beispiel 4.2 gemessene Ausbeute an Monoaromaten mit unterschiedlichen Lösungsmittelgemischen, bestehend aus Methanol/Wasser.
Fig. 3 zeigt die in Beispiel 4.2 gemessenen Kohlenstoffanteile in den unterschiedlichen Produktphasen bei Verwendung von unterschiedlichen Lösungsmittelgemischen, bestehend aus Methanol/Wasser.
Fig. 4 zeigt die in Beispiel 5 gemessene Ausbeute an Monoaromaten bei Verwendung von unterschiedlichen Nano-Membranfiltern.
Fig. 5 zeigt die in Beispiel 6 gemessene Ausbeute an Monoaromaten bei Verwendung von unterschiedlichen Sauerstoffpartialdrücken.
Fig. 6 zeigt die in Beispiel 6 gemessenen Kohlenstoffanteile in den unterschiedlichen Produktphasen bei Verwendung von unterschiedlichen Sauerstoffpartialdrücken.
Fig. 7 zeigt die in Beispiel 6 gemessene Ausbeute an Monoaromaten bei Verwendung von unterschiedlichen Reaktionszeiten.
Fig. 8 zeigt die in Beispiel 6 gemessenen Kohlenstoffanteile in den unterschiedlichen Produktphasen bei Verwendung von unterschiedlichen Reaktionszeiten.
Fig. 9 zeigt die in Beispiel 6 gemessene Ausbeute an Monoaromaten bei Verwendung von unterschiedlichen Katalysatormassen.

Die in den nachfolgenden Beispielen und Vergleichsbeispielen verwendeten Chemikalien wurden, falls nichts anderes angegeben, bei folgenden Bezugsquellen bezogen:
Sigma-Aldrich (St. Louis, MO, USA), Carl Roth GmbH + Co. KG (Karlsruhe, DE), Clariant AG (Muttenz, CH), BASF SE (Ludwigshafen, DE), abcr GmbH (Karlsruhe, DE), VWR International GmbH (Darmstadt, DE) oder Merck KGaA (Darmstadt, DE).

### Beispiel 1: Charakterisierung der verwendeten Lignine

In den nachfolgenden Beispielen und Vergleichsbeispielen wurden folgende Lignin- und Lignosulfonat-Sorten verwendet, die bei Folgenden Bezugsquellen bezogen wurden.

Diese wurden auf ihre Elementarzusammensetzung, Molekulargewichtsverteilung und kompositionelle Zusammensetzung analysiert. Die Ergebnisse sind in Tabelle 2 zusammengefasst.

**Tabelle 1: Übersicht über die verwendeten Lignine**

| **Substrat** | **Quelle** | **Biomasse** | **Prozess** | **C [wt%]** | **H [wt%]** | **N [wt%]** | **S [wt%]** |
|---|---|---|---|---|---|---|---|
| 1A | FAU Erlangen | Weichholz | Organosolv | 60,10% | 6,06% | 0,29% | 0,00% |
| 1B | FAU Erlangen | Weichholz | Organosolv | 58,93% | 6,02% | 0,29% | 0,00% |
| 2 | Sigma-Aldrich | Weichholz | Kraft | 52,44% | 5,07% | 0,00% | 2,73% |
| 3 | Sigma-Aldrich | Weichholz | Kraft | 61,66% | 5,73% | 0,47% | 1,82% |
| 4 | FAU Erlangen - Fraunhofer Leuna | Buche | Organosolv | 60,41% | 6,08% | 0,00% | 0,00% |
| 5 | FAU Erlangen - Fraunhofer Leuna | Fichte | Organosolv | 64,13% | 5,92% | 0,00% | 0,00% |
| 6 | LignoPure, Hamburg | Softwood | High Acid Hydrolysis | 61,57% | 5,77% | 0,00% | 0,00% |
| 7 | LignoPure, Hamburg | Hardwood | Lignosulfonate | 30,27% | 5,70% | 0,00% | 6,89% |
| 8 | LignoPure, Hamburg | Softwood | Kraft | 63,16% | 5,85% | 0,00% | 1,92% |
| 9 | Fraunhofer Leuna | Buche | Organosolv | 60,41% | 6,39% | 0,58% | 0,00% |
| 10 | LignoPure, Hamburg | Birke | 2G Biorefinery | 56,24% | 6,45% | 0,73% | 0,24% |
| 11 | LignoPure, Hamburg | Fichte, Wheat Straw | Diluted acid + enzymatic | 57,29% | 6,15% | 0,41% | 0,20% |
| 12 | LignoPure, Hamburg | Buche | Hydrolysis | 57,32% | 6,25% | 0,58% | 0,00% |
| 13 | LignoPure, Hamburg | Annual Wheat Straw | Purified Soda | 59,14% | 5,97% | 0,71% | 1,06% |
| 14 | Lenzing, Tschechien | Softwood | Lignosulfonat-Mg | 26,50% | 7,21% | 0,00% | 4,27% |
| 15 | Lenzing, Österreich | Softwood | Lignosulfonat-Mg | 27,05% | 6,92% | 0,00% | 5,02% |
| 16 | Fraunhofer, CBP Leuna | Buche | Organosolv | 63,61% | 6,00% | 0,28% | 0,00% |
| | | | Fällen von Lignin mittels | | | | |
| | | | Lösungsmittelverdampfen | | | | |
| 17 | Lixea, Schweden | Weichholz | IL-Fraktioniert | 65,35% | 5,48% | 0,27% | 0,35% |
| 18A | Essity - InnoLig Powder | Strohabfall | Essity-Verfahren | 32,79% | 4,76% | 0,93% | 0,23% |
| 18B | Essity - InnoLig Fluid | Strohabfall | Essity-Verfahren | 15,46% | 8,03% | 0,53% | 0,12% |
| 19 | LignoBoost Rosenthal | Vermutlich Weichholz | Kraft-ähnlich | | | | |

### 1.1 Elementaranalyse:

Die Elementaranalyse für die Elemente C, H, N und S erfolgte mit Hilfe eines "EuroEA-3000 Elemental Analyzer" mit HEKAtech HT Sauerstoff-Analysator der Firma EuroVector/Hekatech GmbH (Wegberg, DE) gemäß Herstellerangabe. Die Sauerstoffgehalte wurden aus der Differenz berechnet.

### 1.2 Molekulargewichtsverteilung:

Die Ermittlung der Molekulargewichtsverteilung der verwendeten Lignine erfolgte mittels Gel-Permeations-Chromatographie (GPC). Hierfür wurde eine PSS MCX 8x50 mm 5 µm Vorsäule und eine 8x300 mm 5 µm 1 000 A sowie eine 8x300 mm 5 µm 100 000 A Hauptsäule der Firma Agilent, Inc. (Santa Clara, CA, USA) bei 35 Grad verwendet. Als Laufmittel wurde eine 0.1 N NaOH-Lösung bei 0.7 mL/min verwendet.

Als Molmassenstandard wurden engverteilte Molmassenstandards von 180 Da bis 976000 Da auf Basis von Na-Benzensulfonat- und entsprechenden Na-Polystyrensulfonat-Salzen verwendet.

Die Ergebnisse sind in Tabelle 2 zusammengefasst.

**Tabelle 2: Molekulargewichtsverteilung der verwendeten Lignine**

| **Substrat:** | **Mn:** | **Mw:** | **D:** |
|---|---|---|---|
| 1A | 864 | 2513 | 2,909 |
| 2 | 1260 | 5391 | 4,28 |
| 3 | 1378 | 6177 | 4,483 |
| 4 | 800,4 | 2234 | 2,791 |
| 5 | 633 | 3607 | 3,183 |
| 6 | 475,2 | 66 | 2,339 |
| 7 | 783,4 | 3717 | 4,745 |
| 8 | 1456 | 6680 | 4,587 |
| 9 | 851,5 | 3049 | 3,581 |
| 10 | 843,7 | 7215 | 8,552 |
| 6 | 704,6 | 1613 | 2,29 |
| 12 | 977,2 | 9348 | 9,566 |
| 13 | 1212 | 5910 | 4,876 |
| 16 | 884,3 | 1809 | 2,046 |

### Beispiel 2 - Herstellung der verwendeten Katalysatoren

Folgende Katalysatoren wurden kommerziell erworben:

| | | |
|---|---|---|
| HPMo-0 | H₃PMo₁₂O₄₀ | kommerziell erworben von Sigma Aldrich |
| HPW-0 | H₃PW₁₂O₄₀ | kommerziell erworben von Sigma Aldrich |
| HSiW-0 | H₄SiW₁₂O₄₀ | kommerziell erworben von Sigma Aldrich |

Die folgenden Katalysatoren wurden in Anlehnung an literaturbekannte Verfahren hergestellt

| | | |
|---|---|---|
| HPMo-2 V | H₅PV₂Mo₁₀O₄₀ | Herstellung nach Albert, J. et al. (2014) |
| HPMo-5 V | H₈PV₅Mo₇O₄₀ | Herstellung nach Albert, J. et al. (2014) |

Albert, J. et al. (2014): "Spectroscopic and electrochemical characterization of heteropoly acids for their optimized application in selective biomass oxidation to formic acid"; Green Chem., 2014,16, Seiten 226-237. DOI: 10.1039/c3gc41320a Die folgenden Katalysatoren wurden nach folgenden Vorschriften hergestellt

| | | |
|---|---|---|
| HPMo-3-2 VMn | H₁₀PV₃Mn₂Mo₇O₄₀ | Herstellung nach Raabe et al., (2022) |
| HPMo-1 Mn | H₇PMn₁Mo₁₀O₄₀ | Herstellung nach Raabe et al., (2022) |

Raabe, J.-C. et al. (2022), "Spectroscopic, Crystallographic, and Electrochemical Study of Different Manganese(II)-Substituted Keggin-Type Phosphomolybdates", Chemistry-A European Journal, 2022, 28 (49), e202201084

| | | |
|---|---|---|
| HPMo- Co₁ | H₇PCo₁Mo₁₁O₄₀ | Herstellung nach Raabe et al., (2024,) |
| HPMo- Co₂ | H₁₁PCo₂Mo₁₀O₄₀ | Herstellung nach Raabe et al., (2024) |
| HPMo- Co₃ | H₁₅PCo₃Mo₉O₄₀ | Herstellung nach Raabe et al., (2024) |

Raabe, J.-C. et al. (2024), "Synthesis and Characterization of Co(II) Substituted Keggin-Type Polyoxometalates as Novel Catalysts for the Hydroformylation of 1-Hexene in a Thermomorphic Solvent System", ChemCatChem, 2024, e202400395.

| | | |
|---|---|---|
| NaPW- Co₃ | NaₓH₍₁₅₋ₓ₎PCo₃W₉O₄₀ | Herstellung nach Raabe et al., (2023) |
| NaPMo- Co₁ | NaₓH₍₇₋ₓ₎PCo₁Mo₁₁O₄₀ | Herstellung nach Raabe et al., (2023) |
| HPMo- Ni₁ | H₇PNiMo₁₁O₄₀ | Herstellung nach Raabe et al., (2023) |
| HPMo- Ni₂ | H₁₁PNi₂Mo₁₀O₄₀ | Herstellung nach Raabe et al., (2023) |
| HPMo- Ni₃ | Hi₅PNi₃Mo₉O₄₀ | Herstellung nach Raabe et al., (2023) |
| NaPMo-Nb₃ | Na₆PNb₃Mo₉O₄₉ | Herstellung nach Raabe et al., (2023) |
| NaPMo-In₄ | Na₁₅PIn₄Mo₈O₄₀ | Herstellung nach Raabe et al., (2023) |
| HPMo-Ni₁Co₁ | H₁₁PNiCoMo₁₀O₄₀ | Herstellung nach Raabe et al., (2023) |

Raabe, J.-C. et al. (2023), "Study on the incorporation of various elements into the Keggin lacunary-type phosphomolybdate [PMo9O34]9- and subsequent purification of the polyoxometalates by nanofiltration", Inorg. Chem. Front., 2023, 10, 4854-4868.

### Beispiele 3.1 bis 3.12 und Vergleichsbeispiele V1 bis V7 - Umsetzung mit verschiedenen Katalysatoren

In den erfindungsgemäßen Beispielen 3.1 bis 3.12 wurden 500 mg des Substrats 4 jeweils mit 200 mg der in Tabelle 4 aufgeführten Katalysatoren in 10 ml Methanol/Wasser in unterschiedlicher volumetrischer Zusammensetzung gelöst und in einem Druckreaktor bei einem Sauerstoffpartialdruck von 14 bar Initialdruck bei Raumtemperatur für 24 h bei einer Temperatur von 140°Cprozessiert.

In den Vergleichsbeispielen V2 bis V7 wurden ebenfalls 500 mg des jeweiligen Substrats jeweils mit 200 mg der in Tabelle 4 aufgeführten Katalysatoren in 10 ml Methanol/Wasser in unterschiedlicher volumetrischer Zusammensetzung gelöst und in einem Druckreaktor bei einem Sauerstoffpartialdruck von 14 bar Initialdruck bei Raumtemperatur für 24 h bei einer Temperatur von 140°C prozessiert.

Zusätzlich wurde in Vergleichsbeispiel 1 als Kontrolle ("Blank") zusätzlich 10 ml Methanol/Wasser in unterschiedlicher volumetrischer Zusammensetzung ohne Katalysator-Zugabe ebenfalls mit 500 mg des Substrats 4 und analogprozessiert. Die Zusammensetzung der gebildeten Monoaromaten ist ebenfalls in Tabelle 6 gezeigt.

### 3.13 Analysemethode:

Die Analyse der während der Umsetzung gebildeten Monoaromaten erfolgte nach Ablauf der 24 h mittels Gaschromatographie mit Massenspektrometrie-Kopplung (GC-MS) unter Verwendung der unpolaren Säule ShinCarbon ST Micropacked GC Column, 2 m Länge, 0,53 mm Innendurchmesser der Firma Restek Corporation (Bellefonte, PA, USA).

Die Gasphase wurde mit Hilfe eines Varian 450-GC der Firma Bruker Corporation (Billerica, MA, USA) mit folgenden Einstellungen analysiert.

Injektionstemperatur: 220°C, Temperatur TCD-Filament: 250°C,

### 3.14 Zusammenfassung der Ergebnisse

**Tabelle 6: Ergebnisse der katalytischen Umsetzung mit verschiedenen Katalysatoren**

| | **Katalytisches System** | **Ausbeute Monoaromaten / Gew.-%** | | | |
|---|---|---|---|---|---|
| Lfd. Nummer | | **Vanillin** | **Methylvanillat** | **Syringaldehyd** | **Methylsyringat** |
| V1 | Blank | 0,60% | 0,34% | 0,43% | 0,37% |
| V2 | HPMo-0 | 0,48% | 1,62% | 0,48% | 1,64% |
| V3 | HPW-O | 0,59% | 1,20% | 0,60% | 1,38% |
| V4 | HSiW-0 | 0,55% | 1,22% | 0,58% | 1,39% |
| V5 | HPMo-2 V | 0,27% | 0,93% | 0,30% | 0,30% |
| V6 | HPMo-5 V | 0,45% | 0,96% | 0,31% | 0,52% |
| V7 | HPMo-3-2 VMn | 0,38% | 0,64% | 0,36% | 0,60% |
| 3.1 | HPMo-1 Co | 1,19% | 2,10% | 1,13% | 2,46% |
| 3.2 | HPMo-2 Co | 1,32% | 1,89% | 1,69% | 2,67% |
| 3.3 | HPMo-3 Co | 1,24% | 1,55% | 1,98% | 2,68% |
| 3.4 | NaPW-3 Co | 0,78% | 0,39% | 1,24% | 0,69% |
| 3.5 | NaPMo-1 Co | 1,08% | 0,77% | 1,37% | 0,96% |
| 3.6 | HPMo-1 Ni | 1,16% | 1,94% | 1,25% | 2,44% |
| 3.7 | HPMo-2 Ni | 1,33% | 2,04% | 1,39% | 2,91% |
| 3.8 | HPMo-3 Ni | 1,41% | 1,75% | 2,19% | 2,92% |
| 3.9 | HPMo-3 Nb | 0,90% | 0,65% | 0,60% | 0,59% |
| 3.10 | HPMo-4 In | 0,73% | 1,54% | 0,76% | 1,25% |
| 3.11 | HPMo-1-1 NiCo | 1,39% | 2,09% | 1,53% | 2,86% |
| 3.12 | HPMo-1 Mn | 0,88% | 1,67% | 0,79% | 1,79% |

Für die weiteren Versuche wurde der Katalysator HPMo-Ni3 verwendet, da dieser Katalysator, wie in Beispiel 3.8 gezeigt, die höchste Ausbeute an den untersuchten Monoaromaten zeigte.

### Beispiel 4 - Vergleich der verwendeten Lösungsmittelsysteme

### 4.1 Auswahl des Lösungsmittelsystems unter Verwendung des Referenzkatalysators HPMo-5 V (H₈PV₅Mo₇O₄₀)

Zunächst wurden 500 mg des Substrats 4 jeweils mit 200 mg des Referenzkatalysators HPMo-5 V in 10 ml der nachfolgend angegebenen Lösungsmittel gelöst und in einem Druckreaktor bei einem initialen Sauerstoffpartialdruck von 14 bar bei Raumtemperatur für 24 h bei einer Temperatur von 140°Cprozessiert.

Als Lösungsmittel wurden Methanol ("MeOH"), ein Methanol-Wasser-Gemisch im Volumenverhältnis 1:1 ("MeOH/H₂O"), Ethanol ("EtOH") und ein Ethanol-Wasser-Gemisch im Volumenverhältnis 1:1 ("EtOH/H₂O") verwendet.

Die Analyse der während der Umsetzung gebildeten Monoaromaten erfolgte nach Ablauf der 24 h mit der in Beispiel 3.13 beschriebenen Methode.

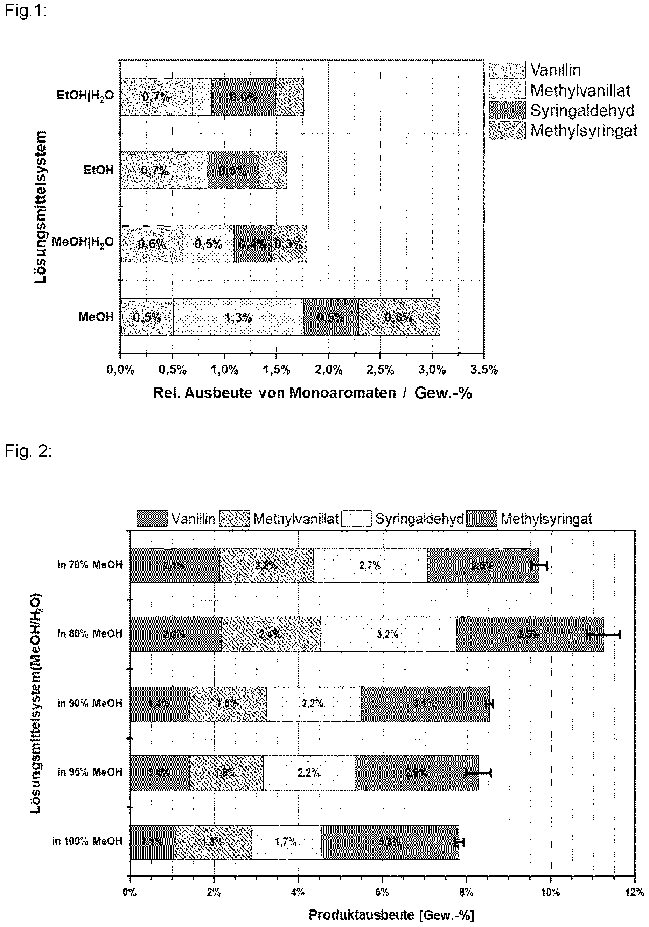

Die Ergebnisse sind in Fig. 1 dargestellt.

### 4.2 Optimierung des Lösungsmittelsystems für den in Beispiel 3.8 ausgewählten Katalysator HPMo-Ni3 (H₁₅PNi₃Mo₉O₄₀).

Zunächst wurden 500 mg des Substrats 9 jeweils mit 200 mg des HPMo-Ni3 (H₁₅PN₁₃Mo₉O₄₀) Katalysators in 10 ml der nachfolgend angegebenen Lösungsmittel gelöst und in einem Druckreaktor bei einem initialen Sauerstoffpartialdruck von 14 bar bei Raumtemperatur für 24 h bei einer Temperatur von 140°C prozessiert.

Folgende Lösungsmittel bzw. Lösungsmittelgemische wurden verwendet:
- 100 Vol.-Methanol ("100% MeOH%),
- 95 Vol.-% Methanol und 5 Vol.-% Wasser ("95% MeOH")
- 90 Vol.-% Methanol und 10 Vol.-% Wasser ("90% MeOH")
- 80 Vol.-% Methanol und 20 Vol.-% Wasser ("80% MeOH")
- 70 Vol.-% Methanol und 30 Vol.-% Wasser ("70% MeOH")

Die Analyse der während der Umsetzung gebildeten Monoaromaten erfolgte nach Ablauf der 24 h mit der in Beispiel 3.13 beschriebenen Methode. Die Ergebnisse sind in Fig. 2 dargestellt.

### 4.2.1 Bestimmung des Kohlenstoffanteils in den verschiedenen Produktphasen.

Weiterhin wurde der Kohlenstoffanteil der während der Reaktion gebildeten Gasphase, der Flüssigphase und der Festphase untersucht.

Analytik der Gasphase erfolgte mittels Gaschromatographie wie oben unter 3.13 beschrieben. Die Analytik der Festphase erfolgte mittels CHNS-Elementanalyse wie oben unter 1.3 beschrieben.

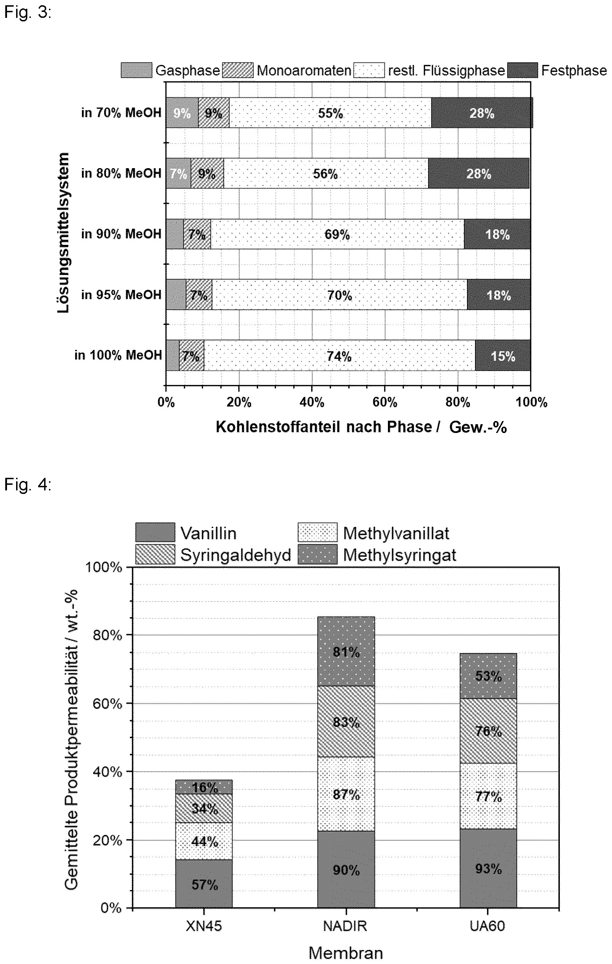

Der Anteil des Kohlenstoffs in der Flüssigphase wurde berechnet, wobei die Einwaage und das Ergebnis der Elementaranalyse als 100% gesetzt wurde und davon der Anteil der entsprechenden Produkte (Monoaromaten) und Phasen (Gasphase und Flüssigphase) abgezogen wurde. Die Ergebnisse sind in Fig. 3 dargestellt.

### Beispiel 5 - Abtrennung der erhaltenen Monoaromaten

Die Membrantrennung erfolgte mit Hilfe des Moduls MiniMem der Firma PS Prozesstechnik GmbH (Basel, CH) mit einer aktiven Membranfläche von 33 cm².

Hierbei wurde ein Feed-Strom von 15 mL/min bei einem Druck von 30 bar und einer Temperatur von 25 °C eingestellt.

Als Nano-Membranfilter wurden folgende Membranfilter der Firma Mann + Hummel (Ludwigsburg, DE) verwendet:
- Polypiperazin-basierte Membran TRISEPO UA60, mit einer Porengrößenverteilung im Bereich von 1000 Dalton,
- Polypiperazin-basierte Membran TRISEPO XN45, mit einem Molekulargewichts-Cut-off im Bereich von 300 bis 500 Dalton, sowie
- Polyethersulfon (PES) basierte Membran NADIRO NP030 P mit einem Molekulargewichts-Cut-off im Bereich von 500 bis 600 Dalton.

### Die Ergebnisse sind in Fig. 4 dargestellt.

Für die Membrantrennung wurden Membranen unterschiedlichen Materials und Porengröße von mehreren Herstellern getestet. Die Membran "NADIR" von MANN+HUMMEL zeigte einen hervorragenden Katalysator-Rückhalt mit >99 Gew.- % und eine Produktpermeabilität von 85 Gew.-%.

### Beispiel 6: Optimierung des kontinuierlichen Prozesses

### 6.1 Optimierung des Sauerstoffpartialdrucks (angelegter Initialdruck vor Aufheizen)

Dazu wurden 500 mg des Substrats 1a jeweils mit 200 mg des Katalysators HPMo-Ni3 (H₁₅PN₁₃Mo₉O₄₀) in 10 ml eines Lösungsmittelgemisches aus 80 Vol.-% Methanol und 20 Vol.-% Wasser gelöst und in einem Druckreaktor bei dem angegebenen initialen Sauerstoffpartialdruck von 5 bar, 14 bar, oder 27,5 bar für 24 h bei einer Temperatur von 140°Cprozessiert.

Die Analyse der während der Umsetzung gebildeten Monoaromaten erfolgte nach Ablauf der 24 h mit der in Beispiel 3.13 beschriebenen Methode.

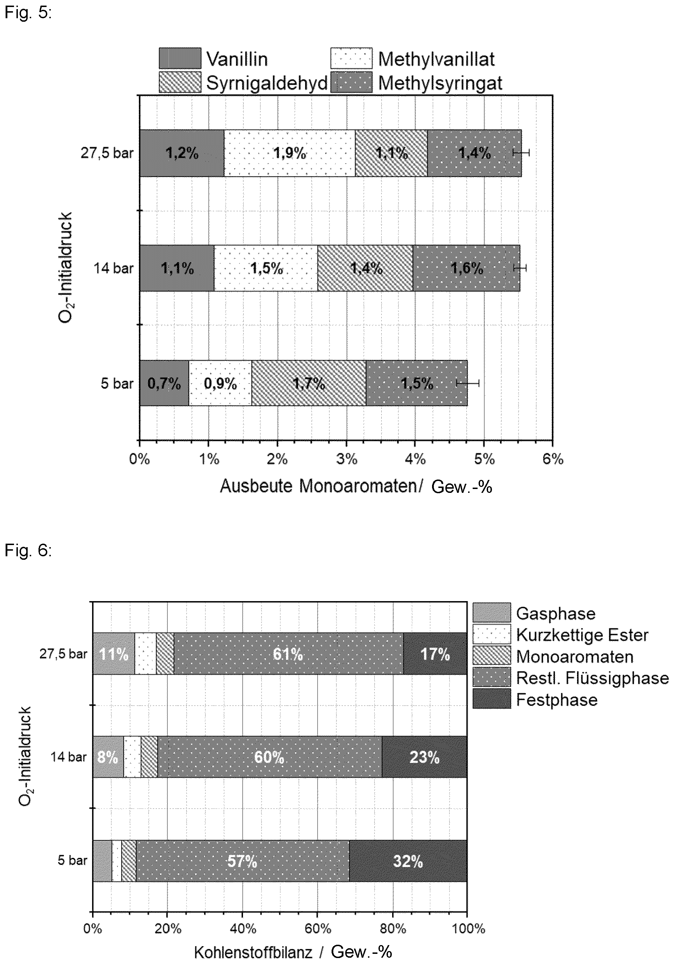

Die Ergebnisse sind in Fig. 5 dargestellt.

Weiterhin wurde der Kohlenstoffanteil der während der Reaktion gebildeten Gasphase, der Flüssigphase und der Festphase, wie oben unter 4.2.1 beschrieben, untersucht. Die Ergebnisse sind in Fig. 6 dargestellt.

### 6.2 Optimierung der Reaktionszeit

Dazu wurden 500 mg des Substrats 1a jeweils mit 200 mg des Katalysators HPMo-Ni3 (H₁₅PN₁₃Mo₉O₄₀) in 10 ml eines Lösungsmittelgemisches aus 80 Vol.-% Methanol und 20 Vol.-% Wasser gelöst und in einem Druckreaktor bei einem initialen Sauerstoffpartialdruck von 14 bar für die Zeiträume von 2 h, 4 h, 6 h, 8 h, 10 h, 12 h, 14 h, 16 h, 18 h, 20 h oder 24 h bei einer Temperatur von 140°C prozessiert.

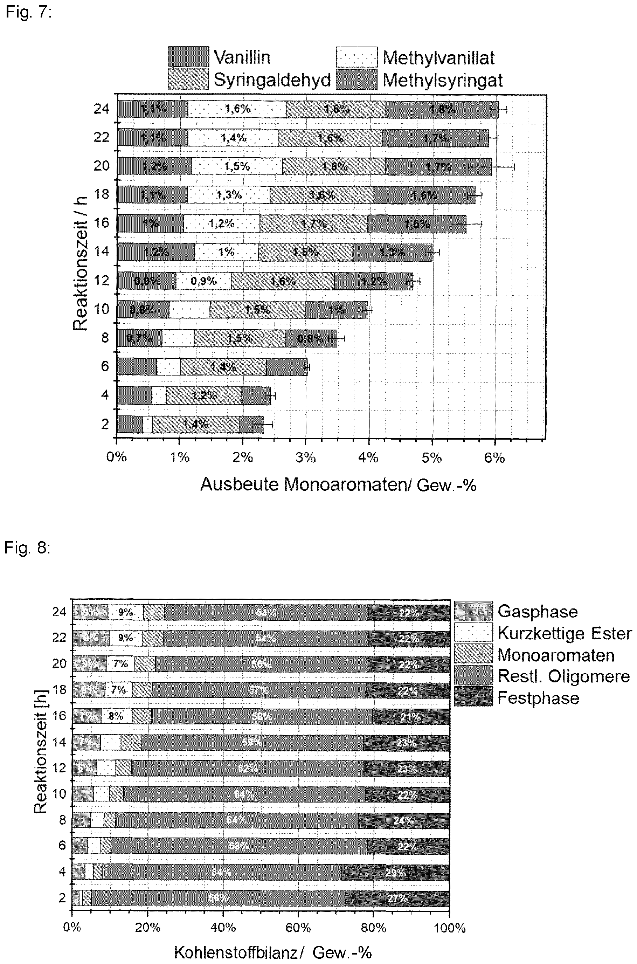

Die Analyse der während der Umsetzung gebildeten Monoaromaten erfolgte nach Ablauf der 24 h mit der in Beispiel 3.13 beschriebenen Methode. Die Ergebnisse sind in Fig. 7 dargestellt.

Weiterhin wurde der Kohlenstoffanteil der während der Reaktion gebildeten Gasphase, der Flüssigphase und der Festphase, wie oben unter 4.2.1 beschrieben, untersucht. Die Ergebnisse sind in Fig. 8 dargestellt.

### 6.2 Optimierung der Katalysatormasse

Dazu wurden 1500 mg des Substrats 1a jeweils mit 100 mg, 150 mg, 200 mg, 300 mg, oder 375 mg des Katalysators HPMo-Ni3 (H₁₅PN₁₃Mo₉O₄₀) in 10 ml eines Lösungsmittelgemisches aus 80 Vol.-% Methanol und 20 Vol.-% Wasser gelöst und in einem Druckreaktor bei einem initialen Sauerstoffpartialdruck von 14 bar für einen Zeiträume von 16 h bei einer Temperatur von 159°Cprozessiert.

Die jeweils verwendete Menge an Substrat und Katalysator resultiert in einem Substrat zu Katalysator-Verhältnis von 15:1, 10:1, 7,5:1, 5:1 und 4:1.

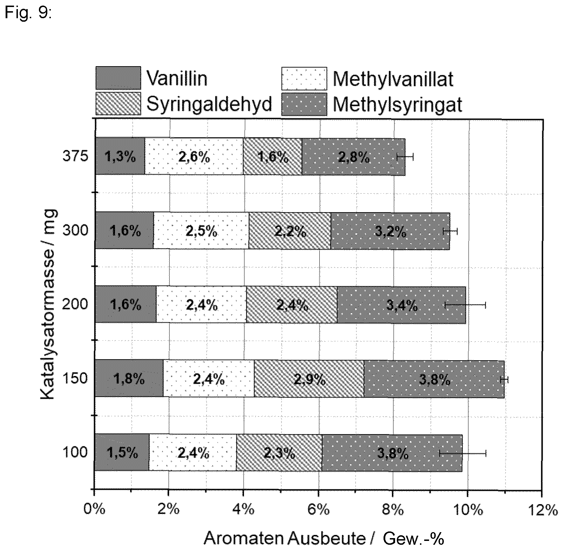

Die Analyse der während der Umsetzung gebildeten Monoaromaten erfolgte nach Ablauf der 16 h mit der in Beispiel 3.13 beschriebenen Methode. Die Ergebnisse sind in Fig. 9 dargestellt.

Es zeigte sich, dass ein Substrat zu Katalysator-Verhältnis von 10:1 (1500 mg Substrat zu 150 mg Katalysator) und eine hohe Temperatur die Ausbeute signifikant verbessern. Die Monoaromatenausbeute konnte so von 5,5 Gew.-% auf 11 Gew.-% verdoppelt werden

## Patentansprüche

1. Verfahren zur Herstellung von Monoaromat-Derivaten durch oxidative Depolymerisierung von Lignin, wobei das Verfahren folgende Schritte umfasst:
(1a) Bereitstellen einer Lignin-haltigen Zusammensetzung,
(1b) Kontaktieren der Lignin-haltigen Zusammensetzung mit Sauerstoff in Gegenwart wenigstens eines Polyoxometallat - Katalysators mit der allgemeinen Formel (I):
Aₙ[XY_{y}Z_{z}MₘO₄₀] (I)
wobei A ausgewählt ist aus Wasserstoff, Alkalimetall, oder Ammonium, wobei n eine ganze Zahl aus einem Bereich von 6 bis 15 bedeutet, und
wobei X ein Heteroatom bedeutet, das ausgewählt ist aus Phosphor oder Silizium,
wobei Y und Z jeweils voneinander verschieden sind und ein Metallatom bedeuten, das jeweils unabhängig voneinander ausgewählt ist aus der Gruppe, die aus Nickel (Ni), Niob (Nb), Indium (In) und Cobalt (Co), besteht, und
wobei M ein Gerüstatom bedeutet, das ausgewählt ist aus der Gruppe, die aus Molybdän (Mo), Wolfram (W) und Kombinationen davon besteht,
wobei der Index m eine ganze Zahl aus einem Bereich von 6 bis 11 bedeutet, wobei der Index y eine ganze Zahl aus einem Bereich von 0 bis 4 bedeutet, und wobei der Index z eine ganze Zahl aus einem Bereich von 1 bis 4 bedeutet bedeuten, mit der Maßgabe, dass die Summe der Indices m, y und z gleich 12 ist,
wobei das Kontaktieren der Lignin-haltigen Zusammensetzung mit Sauerstoff in Gegenwart des wenigstens einen Polyoxometallat - Katalysators in einem Alkohol-haltigen Lösungsmittel bei einer Temperatur von unter 160 °C in Gegenwart von Sauerstoff mit einem Sauerstoffpartialdruck von höchstens 30 bar erfolgt, und
(1c) Abtrennen der gebildeten Monoaromat-Derivate,
wobei die Monoaromat-Derivate ausgewählt sind aus der Gruppe, die aus Vanillin, Isovanillin, ortho-Vanillin, Acetovanillon, Vanillinsäure-Alkylester, Syringaldehyd, Syringasäure-Alkylester, Acetosyringon, Hydroxybenzaldehyd, Hydroxybenzoesäure-Alkylester, 4-Hydroxyacetophenon und Mischungen davon besteht.

2. Verfahren nach Anspruch 1, wobei das Kontaktieren in Schritt (1b) bei einer Temperatur aus einem Bereich von 50°C bis unter 160°C, vorzugsweise von 60°C bis 150°C, erfolgt.

3. Verfahren nach einem der Ansprüche 1 oder 2, wobei das Kontaktieren in Schritt (1b) bei einem Sauerstoffpartialdruck aus einem Bereich von 1 bar bis 30 bar, vorzugsweise von 5 bar bis 28 bar, erfolgt.

4. Verfahren nach einem der Ansprüche 1 bis 3, wobei das Alkohol-haltige Lösungsmittel wenigstens einen aliphatischen Alkohol mit 1 bis 3 Kohlenstoff-Atomen, weiter bevorzugt 1 bis 2 Kohlenstoff-Atomen, und 1 OH-Gruppe, umfasst.

5. Verfahren nach Anspruch 4, wobei der aliphatische Alkohol aus der Gruppe, die aus Methanol, Ethanol, 1-Propanol, und Mischungen, vorzugsweise Methanol, Ethanol und Mischungen davon, davon besteht, ausgewählt ist.

6. Verfahren nach einem der Ansprüche 1 bis 5, wobei das Alkohol-haltige Lösungsmittel weiterhin Wasser in einem Anteil von mindestens 0,5 Vol.-% bis höchstens 40 Vol.-%, vorzugsweise von mindestens 5 Vol.-% bis höchstens 35 Vol.- %, jeweils bezogen auf das Gesamtvolumen des Alkohol-haltigen Lösungsmittels, aufweist.

7. Verfahren nach einem der Ansprüche 1 bis 6, wobei die in Schritt (1a) bereitgestellte Lignin-haltige Zusammensetzung wenigstens ein Organosolv-Lignin, wenigstens ein Kraft-Lignin und/oder wenigstens ein Lignosulfonat umfasst.

8. Verfahren nach einem der Ansprüche 1 bis 7, wobei das Kontaktieren der Lignin-haltigen Zusammensetzung mit Sauerstoff in Gegenwart des wenigstens einen Polyoxometallat - Katalysators in Schritt (1b) für wenigstens 0,5 h, vorzugsweise von 2 h bis 36 h, weiter bevorzugt von 6 h bis 24 h, erfolgt

9. Verfahren nach einem der Ansprüche 1 bis 8, wobei das Kontaktieren der Lignin-haltigen Zusammensetzung mit Sauerstoff in Gegenwart des wenigstens einen Polyoxometallat - Katalysators in Schritt (1b) ohne Verwendung eines Lösungspromoters und/oder Beschleunigungspromotors, wie vorzugsweise Natronlauge, Nitrobenzol, Schwefelsäure, Salzsäure, Salpetersäure, Kupfersulfat oder Toluolsulfonsäure, erfolgt.

10. Verfahren nach einem der Ansprüche 1 bis 9, wobei das Abtrennen der gebildeten Monoaromat-Derivate in Schritt (1c) durch Membrantrennung unter Verwendung wenigstens einer Nanofiltrationsmembran erfolgt.

11. Verfahren nach einem der Ansprüche 1 bis 10, wobei das in Schritt (1c) abgetrennte Monoaromat-Derivat ausgewählt ist aus der Gruppe, die aus Vanillin, Isovanillin, ortho-Vanillin, Acetovanillon, Vanillinsäure-C1-C3-Alkylester, Syringaldehyd, Syringasäure-C1-C3-Alkylester, Acetosyringon und Mischungen davon, vorzugsweise Vanillin, Vanillinsäure-methylester, Vanillinsäure-ethylester, Vanillinsäure-n-propylester Syringaldehyd, Syringasäure-methylester, Syringasäure-ethylester, Syringasäure-n-propylester, und Mischungen davon, besteht.

12. Polyoxometallat - Katalysator mit der allgemeinen Formel (I):
Aₙ[XY_{y}Z_{z}MₘO₄₀] (I)
wobei A ausgewählt ist aus Wasserstoff, Alkalimetall, oder Ammonium, wobei n eine ganze Zahl aus einem Bereich von 6 bis 15 bedeutet, und
wobei X ein Heteroatom bedeutet, das ausgewählt ist aus Phosphor oder Silizium,
wobei Y und Z jeweils voneinander verschieden sind und ein Metallatom bedeuten, das jeweils unabhängig voneinander ausgewählt ist aus der Gruppe, die aus Nickel (Ni), Niob (Nb), Indium (In) und Cobalt (Co) besteht,
wobei der Index m eine ganze Zahl aus einem Bereich von 6 bis 11 bedeutet, wobei der Index y eine ganze Zahl aus einem Bereich von 0 bis 4 bedeutet, und wobei der Index z eine ganze Zahl aus einem Bereich von 1 bis 4 bedeutet bedeuten, mit der Maßgabe, dass die Summe der Indices m, y und z gleich 12 ist.

13. Polyoxometallat - Katalysator nach Anspruch 12, wobei der Polyoxometallat - Katalysator mit der allgemeinen Formel (I) aus der Gruppe, die aus H₁₅PCo₃Mo₉O₄₀, H₁₁PCo₂Mo₁₀O₄₀, H₁₅PNi₃Mo₉O₄₀, H₁₁PNi₂Mo₁₀O₄₀, H₁₁PNiCoMo₁₀O₄₀ und Mischungen davon besteht, ausgewählt ist.

14. Verfahren nach einem der Ansprüche 1 bis 11, wobei der in Schritt (1b) verwendete wenigstens eine Polyoxometallat - Katalysator ein Polyoxometallat - Katalysator nach einem der Ansprüche 12 bis 13 ist.

15. Verwendung eines Polyoxometallat - Katalysators mit der allgemeinen Formel (I):
Aₙ[XY_{y}Z_{z}MₘO₄₀] (I)
wobei A ausgewählt ist aus Wasserstoff, Alkalimetall, oder Ammonium, wobei n eine ganze Zahl aus einem Bereich von 6 bis 15 bedeutet, und
wobei X ein Heteroatom bedeutet, das ausgewählt ist aus Phosphor oder Silizium,
wobei Y und Z jeweils voneinander verschieden sind und ein Metallatom bedeuten, das jeweils unabhängig voneinander ausgewählt ist aus der Gruppe, die aus Nickel (Ni), Niob (Nb), Indium (In) und Cobalt (Co) besteht, und
wobei M ein Gerüstatom bedeutet, das ausgewählt ist aus der Gruppe, die aus Molybdän (Mo), Wolfram (W) und Kombinationen davon besteht, und
wobei der Index m eine ganze Zahl aus einem Bereich von 6 bis 11 bedeutet, wobei der Index y eine ganze Zahl aus einem Bereich von 0 bis 4 bedeutet, und wobei der Index z eine ganze Zahl aus einem Bereich von 1 bis 4 bedeutet bedeuten, mit der Maßgabe, dass die Summe der Indices m, y und z gleich 12 ist,
zur Herstellung von Monoaromat-Derivaten durch oxidative Depolymerisierung von Lignin.
